(19) Europäisches Patentamt
European Patent Office
Office européen des brevets

(11) Veröffentlichungsnummer: **0 341 527 A2**

(12) # EUROPÄISCHE PATENTANMELDUNG

(21) Anmeldenummer: **89107846.1**

(51) Int. Cl.4: **A23K 1/16 , A61K 31/35**

(22) Anmeldetag: **29.04.89**

Claims for the following Contracting States: ES + GR.

(30) Priorität: **07.05.88 DE 3815718**

(43) Veröffentlichungstag der Anmeldung: **15.11.89 Patentblatt 89/46**

(84) Benannte Vertragsstaaten: **AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Scheuermann, Stefan Eugen, Dr.**
**Im Langzahl 23**
**D-6274 Hünstetten(DE)**

(54) **Mittel zur Wachstumssteigerung bei Tieren.**

(57) Die vorliegende Erfindung betrifft Mittel zur Steigerung der Lebendmassezunahme oder verbesserten Futterverwertung oder zu beidem bei mono- oder polygastrischen Tieren, gekennzeichnet durch einen Gehalt an Forskolin oder einem seiner Analoga.

EP 0 341 527 A2

## Mittel zur Wachstumssteigerung bei Tieren

Die Erfindung betrifft das Gebiet der Mittel zur Verbesserung des Wachstums und der Futterverwertung bei Tieren, insbesondere Nutztieren.

Forskolin (I)

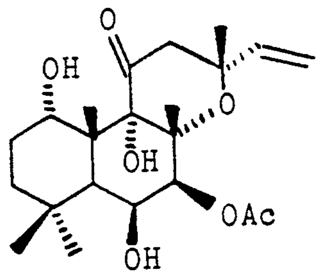

(I)

und seine natürlich vorkommenden Analoga, wie beispielsweise 1,9-Didesoxy-, 9-Desoxy-, 1,9-Didesoxy-7-deacetyl-, 1-Desoxy-, 7-Deacetyl- und 6-Acetyl-7-deacetyl-Forskolin, stellen eine Gruppe von Labdan-Terpenoiden dar, die aus der Pflanze Coleus forskohlii (Briqu.) isoliert werden. Forskolin ist eines der wichtigen Diterpene der Pflanze Coleus forskohlii und kann als wertvolles Arzneimittel bei der Behandlung verschiedener Erkrankungen, wie Glaukom, Hypertonie, Herzinsuffizienz, Entzündungen, Allergien und allgemein Erkrankungen, die durch Störung der cAMP-Produktion hervorgerufen werden, verwendet werden (siehe Tetrahedron Letters 19, 1669 (1977), J. Med. Chem. 26, 486 (1983)). Neben Forskolin und den genannten natürlichen Derivaten sind auch eine Reihe strukturell verwandter Labdan-Derivate bekannt geworden, die eine verwandte biologische Wirkung zeigen; siehe z.B. DE-A-35 02 686. Es wurde nun gefunden, daß Forskolin und seine Analoga über ihre Eigenschaften als Therapeutikum zur Behandlung von Krankheiten hinaus vorteilhaft im landwirtschaftlichen Bereich eingesetzt werden können.

Gegenstand der Erfindung ist die Verwendung von Forskolin und seinen Analoga zur Steigerung der Lebendmassezunahme oder verbesserten Futterverwertung oder zu beidem bei mono-oder polygastrischen Tieren.

Von besonderem Interesse ist dabei die Anwendung bei landwirtschaftlichen Nutztieren männlichen und weiblichen Geschlechts sowie kastrierten Tieren, wie Masthähnchen (Broilern), Mastputen, Ferkeln, Mastschweinen, Kälbern und Mastrindern. Aber auch andere Tiere, wie Kaninchen oder Fische sind geeignet.

Als dem Forskolin analoge Verbindung können strukturell verwandte natürliche oder synthetische Labdan-Derivate mit ähnlicher biologischer Wirkung eingesetzt werden.

Der Wirkstoff wird den Tieren vorzugsweise in einer Dosierung von 0,01 bis 40 mg pro kg Körpermasse und Tag verabreicht. Der Wirkstoff kann auf vielfältige Art und Weise den Tieren verabreicht werden.

Gegenstand der Erfindung sind deshalb auch die Mittel zur Steigerung der Lebendmassezunahme oder verbesserten Futterverwertung oder zu beidem bei mono- oder polygastrischen Tieren, gekennzeichnet durch einen Gehalt an Forskolin oder einem seiner Analoga.

Es ist beispielsweise möglich, den Wirkstoff als Reinsubstanz, Rohprodukt, beispielsweise nach der Pflanzenextraktion, oder als wirkstoffhaltige Pflanzenteile oder in einer geeigneten Zubereitung dem Alleinfutter oder Ergänzungsfutter oder auch einem Teil der Tagesration zuzufügen. Das Futter kann dabei in flüssiger oder fester Form vorliegen. Alternativ kann man den Wirkstoff oder eine Wirkstoffzubereitung dem Trinkwasser zusetzen. Auch ist es möglich, den Wirkstoff mit üblichen Hilfsmitteln in eine oral zu applizierende feste oder flüssige galenische Zubereitung zu bringen und als solche zu verabreichen oder dem Futter zuzusetzen. Auch eine parenterale Applikation des Wirkstoffs mit Hilfe eines Implantats ist möglich.

Eine in vielen Fällen bevorzugte Applikationsform des Wirkstoffs ist die Zugabe zum Futter in Form eines Konzentrats (Praemix). Das Konzentrat kann beispielsweise durch Vermischen des Wirkstoffs, des Rohprodukts oder der wirkstoffhaltigen Pflanzenteile mit einem physiologisch verträglichen, festen oder flüssigen Träger hergestellt werden. Als fester Trägerstoff kommen beispielsweise Getreidenebenprodukte, wie Weizennachmehl, Weizenkleie oder entölte Reiskleie, aber auch Maismehl, Sojamehl, Bolus alba oder Calciumcarbonat, in Betracht. Als flüssige Träger können physiologische Salzlösungen, Wasser und physiologisch verträgliche organische Lösungsmittel Verwendung finden. Dabei ist es möglich, geeignete Zusatzstoffe, wie Emulgatoren, Dispersions-, Suspensions-, Benetzungs- oder Geliermittel zu verwenden.

Das Konzentrat (Praemix) enthält in der Regel 0,1 bis 10 Gew.-%, vorzugsweise 0,5 bis 5 Gew.-%, des Wirkstoffs, wobei je nach Anwendungszweck die Wirkstoffkonzentration auch unter-oder überschritten werden kann.

Bei der Verabreichung mit Futtermitteln wird zweckmäßig so verfahren, daß ein Konzentrat mit dem Futter homogen vermengt wird. Bei den Futtermitteln eignen sich die üblicherweise verwendeten Futtermittel, wie beispielsweise verschiedene Getreide, Nachprodukte der Ölgewinnung (z.B. Sojaextraktionsschrot) und andere Energie- und Proteinträger, wie Tapioka und Fischmehl, sowie daraus hergestellte Futtermischungen, Ergänzungsfuttermittel oder Mineralstoffmischungen.

Die optimale Wachstumssteigerung hängt in der Regel von der Zusammensetzung des Futters, insbesondere dem Proteingehalt, und von dem Gehalt an Forskolin bzw. seinen Analoga ab, wobei in der Regel bei steigendem Forskolingehalt ein Maximum der Wachstumssteigerung durchlaufen wird. Ebenso ist zu beobachten, daß die Wachstumssteigerung bei Steigerung des Proteingehalts des Futters ein Maximum durchläuft, wenn man den Wirkstoffgehalt konstant hält. Die optimale Dosierung läßt sich durch eine faktorielle Versuchsplanung in relativ wenigen Vorversuchen leicht ermitteln. In der Regel ist die Konzentration des Wirkstoffes im Futter 0,1 bis 500 mg/kg, vorzugsweise 0,1 bis 200 mg/kg, insbesondere 0,1 bis 100 mg/kg.

Eine weitere Form der Verabreichung des Wirkstoffs besteht in der Lösung oder Suspension im Trinkwasser oder anderen Tränken, wie z.B. dem Milchaustauscher.

Die Verabreichung des Wirkstoffs kann auch in der Weise erfolgen, daß den Tieren, vorzugsweise während oder kurze Zeit vor oder nach der Fütterung, der Wirkstoff in Form seiner festen oder flüssigen galenischen Zubereitung, direkt oral verabreicht wird. Die galenische Zubereitung kann dabei z.B. eine Tablette, Kapsel, Paste, Granulatform, ein Pulver, Bolus, Saft oder Sirup oder ein wie oben beschriebenes Praemix-Konzentrat sein. Inbesondere bei Weidetieren kann diese Applikationsform von besonderem Interesse sein.

Für die Verabreichung in Form von Tabletten, Kapseln, Pasten, Boli, Pillen, Granulaten, Säften, Sirups und ähnlichem können die gleichen Hilfs- und Zusatzstoffe verwendet werden, wie sie in der pharmazeutischen Technik bekannt sind, insbesondere Hilfsstoffe für Zubereitungen zur Langzeitapplikation mit kontinuierlicher Freisetzung des Wirkstoffs. Von besonderem Interesse sind Zubereitungen letzterer Art, die speziell für Wiederkäuer entwickelt worden sind. Der Wirkstoff kann beispielsweise mit pulverförmigen Verdünnungsmitteln, wie z.B. mikrokristalliner Cellulose,. Zucker oder Stärke, zum Abfüllen des Kapselvolumens vermischt werden. Die Herstellung der Tabletten kann ebenfalls auf übliche Weise unter Zugabe von Substanzen, wie z.B. Cellulose, Lactose, Natriumchlorid, Stärke, Dextrin, Cellulosederivaten etc., erfolgen. Auch für die Herstellung von flüssigen Zubereitungen können die in der Pharmazie üblichen Hilfsstoffe, wie z.B. Pflanzenöle, Kollidon, Cellulosederivate und ähnliches verwendet werden. Eine wäßrige Lösung oder Suspension von Forskolin bzw. dessen Analoga kann z.B. neben dem Wirkstoff auch Nebenbestandteile aus dem Rohextrakt der Pflanzen, insbesondere gemahlene Pflanzenteile, oder geeignete Puffersubstanzen enthalten. Pro Verabreichungseinheit soll vorzugsweise 1 bis 600 mg Wirkstoff enthalten sein.

Bei der parenteralen Applikation des Wirkstoffs wird der Wirkstoff vorzugsweise mittels eines in üblicher Weise hergestellten und eingesetzten Implantats je nach Tierart über einige Wochen oder Monate kontinuierlich in der erforderlichen Dosis freigesetzt.

Auch Kombinationen mit anderen Futterzusatzstoffen, wie Antibiotika und Chemotherapeutika sind zur Verbesserung der Wirkung möglich.

Die erfindungsgemäß eingesetzten Wirkstoffe, vorzugsweise Forskolin, beeinflussen das Wachstum bei suboptimaler und optimaler Proteinversorgung positiv. Darüberhinaus ist bei bedarfsgerechter Proteinversorgung in der Regel eine geringere Menge an Futter für die gleiche Gewichtszunahme erforderlich und damit die Futterverwertung gesteigert.

Zusätzlich ist ein proteinsparender Effekt festzustellen. So läßt sich in der Regel mit Forskolin und seinen Analoga im Bereich suboptimaler Proteinversorgung ein bestimmtes Wachstum der Tiere mit einem Futter mit geringerem proteingehalt erreichen als bei einer Fütterung ohne den Wirkstoffzusatz.

**Beispiele**

**1. Fütterungsversuch an männlichen Ratten**

**1a)** Material und Methoden

In einem Fütterungsversuch mit 60 männlichen Ratten im Gewichtsabschnitt von 40 bis 170 g Lebendmasse wurde die Wirkung von 5, 10, 20 und 40 ppm Forskolin im Futter bei zwei verschiedenen Proteingehalten der Diäten (15 und 20 Gew.-% für eine suboptimale bzw. optimale Versorgung) auf das Wachstum, die Futteraufnahme und die Futterverwertung im Vergleich zum Futter ohne Gehalt an Forskolin untersucht. Die Diäten (Zusammensetzung siehe Tabelle 1) waren mit 16,6 und 16,8 kJ/g Trockensubstanz isokalorisch. Jeweils sechs Tiere wurden mit Futter gleichen Gehalts an Forskolin und Protein gefüttert und die Resultate für die Auswertung ermittelt. Die jeweiligen sechs Tiere wurden zu je zweien in Makrolonkäfigen in einer Klimakammer bei konstant 25° C und 65 % relativer Luftfeuchte 28 Tage lang gehalten.

Tabelle 1

Zusammensetzung der Futtermittel für die Versuchsdiäten

| Inhaltsstoffe | Proteingehalt (Gew.-%) | |
|---|---|---|
| | 15 | 20 |
| Glucose | 62,15 | 58,11 |
| Eieralbumin | 15,79 | 21,05 |
| Sojaöl | 8,22 | 7,00 |
| Cellulose | 6,00 | 6,00 |
| *) Mineralstoffvormischung | 5,64 | 5,64 |
| *) Spurenelementvormischung | 0,20 | 0,20 |
| *) Vitaminvormischung | 2,00 | 2,00 |
| Energiegehalt kJ/g T.S. | 16,6 | 16,8 |

*) Zusammensetzung der Vormischungen nach Scheuermann u. Lantzsch, 1982, Z. Tierphys. Tierernähr. u. Futtermittelkde., 48, 224-231

**1b)** Versuchsergebnisse

Die Versuchsergebnisse sind in den Tabellen 2 (Gewichtsentwicklung und Futteraufnahme), 3 (statistische Auswertung) und 4 (Futterverwertung) zusammengefaßt.

Tabelle 2

Gewichtsentwicklung und Futteraufnahme

| Nr. | Gehalt Rohprotein | Gehalt Forskolin (ppm) | Gewicht (g) | | Gewichtszunahme | | Futteraufnahme | |
|---|---|---|---|---|---|---|---|---|
| | | | bei Beginn | am Ende | (g/28 d) | relativ zur Kontrolle (%) | (g/d) | relativ zur Kontrolle |
| 1 | 15 | 0 | 41,0 | 128,7 | 87,7 | 100,0 | 8,11 | 100 |
| 2 | 15 | 5 | 40,4 | 131,3 | 90,9 | 103,6 | 9,07 | 111,9 |
| 3 | 15 | 10 | 40,9 | 156,0 | 115,1 | 131,2 | 10,70 | 132,2 |
| 4 | 15 | 20 | 40,6 | 162,6 | 122,0 | 139,1 | 11,00 | 135,7 |
| 5 | 15 | 40 | 40,5 | 122,3 | 81,8 | 93,3 | 8,26 | 101,8 |
| 6 | 20 | 0 | 41,3 | 135,5 | 94,2 | 100,0 | 9,25 | 100,0 |
| 7 | 20 | 5 | 40,1 | 157,7 | 117,6 | 124,4 | 10,70 | 115,7 |
| 8 | 20 | 10 | 40,3 | 153,2 | 112,9 | 119,9 | 10,40 | 112,4 |
| 9 | 20 | 20 | 41,4 | 161,9 | 120,5 | 127,9 | 10,70 | 115,7 |
| 10 | 20 | 40 | 39,9 | 143,0 | 103,1 | 109,4 | 9,31 | 100,6 |

| Tabelle 3 | | | | | |
|---|---|---|---|---|---|
| Aus Datenmaterial der Tabelle 2 errechnete nicht-lineare Regression ($y = a + bx + cx^2$) für y = Zunahme an Lebendmasse und x = Gehalt Forskolin in der Diät | | | | | |
| Gehalt Rohprotein | Konstanten | | | Korrelationskoeff. R | Signifikanz P ≦ |
| | a | b | c | | |
| 15 | 83,22 | 3,78 | -0,10 | 0,955 | 0,05 |
| 20 | 98,90 | 2,23 | -0,05 | 0,873 | 0,05 |

| Tabelle 4 | | | | | | |
|---|---|---|---|---|---|---|
| Futterverwertung (g Futter/g Gewichtszunahme) relativ zur Kontrollgruppe in % (je niedriger die Zahl desto höher die Futterverwertung) | | | | | | |
| Gehalt Protein (%) | Forskolingehalt (ppm) | | | | | mittlere Futterverwertung |
| | 0 | 5 | 10 | 20 | 40 | |
| 15 | 100 | 107,7 | 100,8 | 97,3 | 109,3 | 103,7 |
| 20 | 100 | 92,4 | 93,8 | 90,5 | 92,0 | 92,2 |

Gewichtsentwicklung:

Bei 15 und 20 Gew.-% Protein zeigt sich bis zu einer Konzentration von 20 ppm Forskolin eine bis zu 39 % gesteigerte Zunahme an Lebendmasse innerhalb von 28 Tagen im Vergleich zur entsprechenden Kontrollgruppe. Es besteht eine signifikante Beziehung zwischen der Gewichtsentwicklung und der Forskolinkonzentration. Zusätzlich wird ein proteinsparender Effekt deutlich. So erreichten die Tiere bei 15 % Protein und 5, 10 oder 20 ppm Forskolin mit ca. 91, 115 oder 122 g Zuwachs die Zuwachsraten der Tiere bei 20 % Protein in der Kontrollgruppe und den Gruppen mit 10 oder 20 ppm Forskolingehalt, entsprechend 94, 113 oder 121 g Zuwachs. Somit ist bei vergleichbarem Wachstum und reduziertem Proteingehalt der Diät die Proteinverwertung in Gegenwart von Forskolin verbessert und die Ausscheidung an Stickstoff über Kot und Harn reduziert.

Futterverwertung:

Die Futterverwertung ist bei bedarfsgerechter (optimaler) Proteinversorgung in Gegenwart von Forskolin erhöht. In der Versuchsreihe ist die Futterverwertung bei 20 % Proteingehalt bis zu etwa 8 % (s. mittlere Futterverwertung in Tabelle 4) erhöht.

Die obengenannten Resultate, die bei den Versuchen an Ratten erhalten wurden, lassen sich in ihrer Tendenz auch auf andere Tiere übertragen. So ist die Verwendung von Ratten bei der Bestimmung der Proteinqualität von Futtermitteln, der limitierenden Aminosäuren und der Proteinverwertung seit langem üblich. Tests an Ratten zur Bestimmung der Proteinverwertung und der biologischen Wertigkeit von Protein in Futtermitteln ist beispielsweise in den USA, Kanada und der Bundesrepublik Deutschland offiziell anerkannt (vgl. bspw. C. Kies, H.M. Fox, Seiten 1 bis 10; J.M. McLaughlan, M.O. Keith, Seiten 79 bis 85; M. Wamack, D.A. Vaughan, C.E. Bodwell, Seiten 113 bis 123; jeweils in "Protein Nutritional Quality of Foods and Feeds, Part 1: Assay Methods - Biological, Biochemical and Chemical; edited by Mendel Friedmann, 1975, Marcel Dekker, New York).

**2. Futterungsversuch an Broilern**

**2a)** Methode

Männliche Lohmann-Masthähnchen mit einer Lebendmasse zwischen 35 und 45 g wurden in Gruppen von jeweils 20 Tieren pro Behandlung (100 $cm^2$/Tier) auf einer Einstreu aus Hobelspänen bei 24-stündiger Beleuchtung und einer Temperatur von 36 ± 1 °C bis zum 21. Versuchstag und 26 ± 1 °C vom 22. bis zum 35. Versuchstag gehalten. Das Futter wurde ad libitum angeboten. Die Zusammensetzung des Futters (ohne Wirkstoff) ist in Tabelle 5 angegeben. Der Proteingehalt betrug 22 Gew.-% (entsprechend dem Bedarf). Forskolin wurde als Wurzelmehl über eine Glucose-Vormischung in die Ration eingebracht.

Tabelle 5

| Tabelle 5 | |
|---|---|
| Futterzusammensetzung (Gew.-% Frischsubstanz) | |
| Sojaextraktionsschrot | 24,00 |
| Fischmehl | 6,00 |
| Futterhefe | 2,00 |
| Mais | 44,89 |
| Weizen | 6,35 |
| Rindertalg | 4,70 |
| Weizennachmehl | 8,50 |
| Luzernegrünmehl | 1,00 |
| phosphors. Futterkalk | 1,40 |
| kohlens. Futterkalk | 0,96 |
| Spurenelementvormischung | 0,04 |
| Viehsalz | 0,09 |
| DL-Methionin | 0,07 |

**2b)** Ergebnisse

Die Versuchsergebnisse sind in Tabelle 6 (s. Seite 12) zusammengefaßt.

Wachstum:

Die Lebendmassezunahme der Broiler war gegenüber der Kontrollgruppe bis zu 9 % verbessert (siehe Tabelle 6).

Futteraufnahme und Futterverwertung:

Die Futteraufnahme wurde durch den Gehalt an Forskolinwurzeln erhöht. Die Erhöhung der Futteraufnahme war jedoch deutlich geringer als die Steigerung in der Lebendmasse, wie auch aus der besseren Futterverwertung (d.h. niedrigere Zahl) zu entnehmen ist. Die Erhöhung der Futteraufnahme deutet außerdem auf eine positive Wirkung auf die Verdauung der Nährstoffe hin, so daß bei einer schnelleren Passage des Chymus mehr Futter aufgenommen werden kann.

Futterverwertung:

Durch die stärkere Steigerung des Wachstums im Vergleich zur Futteraufnahme, wurde die Futterverwertung um bis zu 7 % verbessert (Tabelle 6). Die positiven Effekte auf die Futterverwertung und die Futteraufnahme weisen auf die verbesserte intermediäre Ausnutzung der Futterinhaltsstoffe hin. Dadurch ist auch bei Broilern (vgl. Experiment mit Ratten) mit einer Reduzierung der N-Ausscheidungen zu rechnen.

| Tabelle 6 *) | | | | | | |
|---|---|---|---|---|---|---|
| Gehalt Forskolin (ppm) | Gewicht (Masse) (g) | | Massezunahme (g/d) | Futteraufnahme g/d | Futterverwertung g Futter/g Zunahme |
| | bei Beginn | am Ende | | | |
| 0 | 121,7 ± 17,4 | 1580,2 ± 230,1<br>100,0 | 41,7 ± 6,2<br>100,0 | 84,8<br>100,0 | 2,08 ± 0,34<br>100,0 |
| 2 | 122,6±16,9 | 1715,2±196,2<br>108,5 | 45,4±5,4<br>109,0 | 88,4<br>104,0 | 1,97±0,24<br>94,6 |
| 4 | 126,9±17,2 | 1682,5±253,7<br>106,4 | 44,4±6,9<br>106,0 | 86,2<br>101,6 | 1,99±0,38<br>95,7 |
| 8 | 120,8±17,1 | 1643,0±192,3<br>103,9 | 43,5±5,3<br>104,3 | 83,8<br>98,7 | 1,95±0,24<br>93,8 |
| 16 | 121,6±16,1 | 1629,5±242,4<br>103,1 | 43,1±6,6<br>103,4 | 86,7<br>102,0 | 2,04±0,33<br>97,9 |
| 32 | 122,6±18,6 | 1642,7±182,9<br>103,9 | 43,3±5,0<br>103,9 | 86,4<br>101,8 | 2,02±0,28<br>97,2 |

*) Die erste Reihe in jeder Rubrik bei gleichem Forskolingehalt gibt den gemittelten Wert wieder, die zweite Reihe den Streubereich und die dritte Reihe den prozentualen Wert relativ zur Kontrollgruppe ohne Forskolingehalt im Futter.

## Ansprüche

1. Verwendung von Forskolin und seinen Analoga zur Steigerung der Lebendmassezunahme oder verbesserten Futterverwertung oder zu beidem bei mono- oder polygastrischen Tieren.

2. Mittel zur Steigerung der Lebendmassezunahme oder verbesserten Futterverwertung oder zu beidem bei mono-oder polygastrischen Tieren, gekennzeichnet durch einen Gehalt an Forskolin oder einem seiner Analoga.

3. Mittel nach Anspruch 2, dadurch gekennzeichnet, daß es ein festes oder flüssiges Allein- oder Ergänzungsfutter oder ein Trinkwasser darstellt.

4. Mittel nach Anspruch 2, dadurch gekennzeichnet, daß es ein festes oder flüssiges Konzentrat (Praemix) darstellt.

5. Mittel nach Anspruch 9, dadurch gekennzeichnet, daß es eine oral zu applizierende, feste oder flüssige galenische Zubereitung, vorzugsweise eine Tablette, Kapsel, Paste, ein Granulat, ein Pulver, einen Saft, einen Sirup, einen Bolus oder Slow-Release-Bolus, darstellt.

6. Mittel nach Anspruch 2, dadurch gekennzeichnet, daß es ein Implantat zur parenteralen Applikation von Forskolin mit einer kontinuierlichen Freisetzung über einige Wochen oder Monate darstellt.

7. Mittel nach einem der Ansprüche 2 bis 6, dadurch gekennzeichnet, daß es Forskolin als Reinsubstanz, Rohextrakte, getrocknete Pflanzen oder als Pflanzenteile oder Gemische davon enthält.

8. Mittel nach Anspruch 3, dadurch gekennzeichnet, daß es den Wirkstoff in einer Konzentration von 0,1 bis 500 mg/kg Futter, vorzugsweise 0,1 bis 200 mg/kg Futter, insbesondere 0,1 bis 100 mg/kg Futter, enthält.

9. Mittel nach Anspruch 4, dadurch gekennzeichnet, daß es Forskolin bzw. seine Analoga in einer Konzentration von 0,1 bis 10 Gew.-% enthält.

10. Mittel nach Anspruch 5, dadurch gekennzeichnet, daß es den Wirkstoff in einer Konzentration von 1 bis 600 mg pro Verabreichungseinheit enthält.

11. Verfahren zur Herstellung des Mittels nach Anspruch 2, dadurch gekennzeichnet, daß man Forskolin oder seine Analoga in geeigneter Form mit festen oder flüssigen Futtermitteln oder galenischen Hilfs- und Zusatzstoffen oder mit dem Trinkwasser mischt.

12. Verwendung nach Anspruch 1, dadurch gekennzeichnet, daß man eines oder mehrere der in den Ansprüchen 2 bis 10 definierten Mittel einsetzt.

13. Verwendung nach Anspruch 1 oder 12, dadurch gekennzeichnet, daß die Dosierung 0,01 bis 40 mg Forskolin pro kg Körpermasse der Tiere und pro Tag ist.

Patentansprüche für folgende Vertragsstaaten: ES, GR

1. Verfahren zur Steigerung der Lebendmassezunahme oder verbesserten Futterverwertung oder zu beidem bei mono-oder polygastrischen Tieren, dadurch gekennzeichnet, daß man diesen ein Forskolin oder eines seiner Analoga enthaltendes Mittel verabreicht.

2. Verfahren nach Anspruch 1, dadurch gekennzeichnet, daß das Mittel ein festes oder flüssiges Allein- oder Ergänzungsfutter oder ein Trinkwasser darstellt.

3. Verfahren nach Anspruch 1, dadurch gekennzeichnet, daß das Mittel ein festes oder flüssiges Konzentrat (Praemix) darstellt.

4. Verfahren nach Anspruch 1, dadurch gekennzeichnet, daß das Mittel eine oral zu applizierende, feste oder flüssige galenische Zubereitung, vorzugsweise eine Tablette, Kapsel, Paste, ein Granulat, ein Pulver, einen Saft, einen Sirup, einen Bolus oder Slow-Release-Bolus, darstellt.

5. Verfahren nach Anspruch 1, dadurch gekennzeichnet, daß das Mittel ein Implantat zur parenteralen Applikation von Forskolin mit einer kontinuierlichen Freisetzung über einige Wochen oder Monate darstellt.

6. Verfahren nach Anspruch 1 bis 5, dadurch gekennzeichnet, daß das Mittel Forskolin als Reinsubstanz, Rohextrakte, getrocknete Pflanzen oder als Pflanzenteile oder Gemische davon enthält.

7. Verfahren nach Anspruch 2, dadurch gekennzeichnet, daß das Mittel den Wirkstoff in einer Konzentration von 0,1 bis 500 mg/kg Futter, vorzugsweise 0,1 bis 200 mg/kg Futter, insbesondere 0,1 bis 100 mg/kg Futter, enthält.

8. Verfahren nach Anspruch 3, dadurch gekennzeichnet, daß das Mittel Forskolin bzw. seine Analoga in einer Konzentration von 0,1 bis 10 Gew.-% enthält.

9. Verfahren nach Anspruch 4, dadurch gekennzeichnet, daß das Mittel den Wirkstoff in einer Konzentration von 1 bis 600 mg pro Verabreichungseinheit enthält.

10. Verfahren zur Herstellung des Mittels nach Anspruch 1, dadurch gekennzeichnet, daß man Forskolin oder seine Analoga in geeigneter Form mit festen oder flüssigen Futtermitteln oder galenischen Hilfs- und Zusatzstoffen oder mit dem Trinkwasser mischt.

11. Verwendung von Forskolin und seinen Analoga zur Steigerung der Lebendmassezunahme oder verbesserten Futterverwertung oder zu beidem bei mono- oder polygastrischen Tieren.

12. Verwendung nach Anspruch 11, dadurch gekennzeichnet, daß man eines oder mehrere der in den Ansprüchen 1 bis 9 definierten Mittel einsetzt.

13. Verwendung nach Anspruch 11 oder 12, dadurch gekennzeichnet, daß die Dosierung 0,01 bis 40 mg Forskolin pro kg Körpermasse der Tiere und pro Tag ist.